# EUROPEAN PATENT SPECIFICATION

(11) **EP 3 381 351 B1**
(45) Date of publication and mention of the grant of the patent: **06.05.2026**
(21) Application number: 17163664.0
(22) Date of filing: 29.03.2017
(51) Int. Cl.: A61B 3/113

(54) **SYSTEM FOR ASSESSING A HEALTH CONDITION OF A USER**
SYSTEM ZUR BEURTEILUNG DES GESUNDHEITSZUSTANDS EINES ANWENDERS
SYSTÈME POUR ÉVALUER UN ÉTAT DE SANTÉ D'UN UTILISATEUR

(43) Date of publication of application: 03.10.2018
(73) Proprietor: Carl Zeiss Vision International GmbH, 73430 Aalen (DE)
(72) Inventor: ZAKHAROV, Dr. Pavel, 8604 Volketswil (CH); MROCHEN, Prof. Dr. Michael, 8193 Eglisau (CH)
(74) Representative: Frenkel, Matthias Alexander

(56) References cited:
- KR-A- 20040 023 945
- US-A1- 2016 022 135
- US-A1- 2016 166 204
- US-B1- 9 418 617

## Description

The present invention relates to a system for assessing a health condition of a user and a method for assessing the health condition of the user.

Usually, systems for eye blink detection are applied in the case of sleep warning of drivers. Thereby, a path of light from a source to a detector may be interrupted by a blinking eye and can thus be identified. Further, reflectance based eye tracking systems can detect a viewing direction of a user by triggering an event when the user is looking in a direction of a light source or a light sensor. If the user is looking straight ahead, an incident ray from the light source is substantially reflected from the scattering sclera to give a first level outputted by the light sensor. Next, with the eye turning to look in the direction of the light source, the ray principally strikes the iris, which produces a reduction in the light level outputted by the light sensor. This may cause an electrical output of the light sensor to change significantly and to change a control device such as a relay switch to which it is connected for effecting any desired control activity. Further, a blinking detection technique may be based on a pair of a light source and a detector. Further, reflection intensities between an eyelid and an eyeball can be effectively utilized to provide a device which is not subject to accidental activation due to minor eye movement. For example, with the proper arrangement of light source and detector when the eye is open, most of an incident light will be absorbed by the eyeball, and only a small portion is reflected. When the eye is closed, a greater portion of the incident light is reflected by the eyelids as compared to the eyeball.

US 9,418,617 B1 discloses a comprehensive calibration method for blink detection in head-mounted displays (HMDs) using proximity sensors:
- Proximity sensors detect eye activity (blinks, normal movement, winks) and capture magnitude changes in sensor readings over time (blocks 302-304)
- System generates statistical distribution of magnitude-change values collected during a calibration interval (block 304)
- System identifies clusters within the distribution representing different types of eye activity (block 306) based on frequency of occurrence and magnitude ranges
- System generates reference data mapping clusters to specific eye activity types: normal activity (lower magnitude-change cluster), blinks (higher magnitude-change cluster), winks (highest magnitude-change values) (block 308)
- Distribution is updated continuously or at predefined intervals (e.g., every three minutes) to adapt to changes in HMD orientation, lens shade, or user
- System stores distributions per user account and switches between distributions when different users use the device
- Gaussian distributions, k-means clustering, and expectation-maximization algorithms are used to refine cluster identification and parameter estimation.

It is an object of the present invention to improve a treatment of a patient suffering, for example, from a dry eye phenomenon and medically support the patient.

According to a first aspect, a system for assessing a health condition of a user comprises a sensor unit, a monitoring unit and a storage unit. The sensor unit comprises at least one eye sensor. The at least one eye sensor is adapted to obtain an optical signal reflected from an eye of the user. The sensor unit can be mounted on a wearable device. The monitoring unit is connected to the sensor unit. The monitoring unit is adapted to derive data related to an eye activity of the user by processing the optical signal. The data related to the eye activity of the user is included in the optical signal. The storage unit is connected to the monitoring unit.

The storage unit is adapted to store the derived data related to the eye activity of the user and recorded data. The monitoring unit is further adapted to obtain the recorded data from the storage unit. The monitoring unit is further adapted to assess the health condition of the user by comparing the recorded data with the derived data related to the eye activity of the user.

The sensor unit and the monitoring unit may be connected via a radio channel (e.g. Bluetooth, ANT+, WiFi), optical channel (e.g. LiFi, infrared channel) or digital bus (e.g. USB, I2C, SPI).

The advantage of the system lies in that it provides a tool for supporting a patient with information related to the patient's health condition. The sensor unit enables the patient to acquire data gathering for different users and for himself/herself. This generates a datapool making a patient's decision on a medical behaviour more precise.

The term "eye activity" may be understood as an adjustment of the eye physiological state. This includes blinks, eye movements, such as rotational movement, pupil size changes, accommodation, tear film quality, tear film motion, etc. Further eye activity may be referred to by a parameter of interest. The parameter of interest may be a frequency of blinks (i.e. blinks/minute), interblink interval (i.e. seconds), eye movements (i.e. vertical vs. horizontal), pupil radius, blink completeness and other such as refraction properties.

The wearable device may be adapted to be wearable on the head of the user. The sensor unit may be mounted on the wearable device. The wearable device may be a spectacles frame. This can be a dedicated frame or normal glasses (both prescribed and non-prescribed) suitable to hold a sensor attachment.

The monitoring unit may be a separate device or can also be mounted on the wearable device. The monitoring unit may be further adapted to carry out the processing of (raw) data, evaluating parameters of the eye activity, and controlling an exchange of data between the sensor and the monitoring unit, the sensor and the storage unit and/or the monitoring unit and the storage unit. The monitoring unit may be in the form of a wearable or mobile device, such as a smartphone, tablet, desktop, laptop computer or dashboard software. The monitoring unit may be located on a frame of a wearable device or can be a separate unit. The monitoring unit may comprise a controller adapted to display information to the user via a screen or indicator. The controller may be adapted to accept an input from the user via buttons, gestures (both head, hands' and arms' movements) or via other means. The controller may have additional sensors adapted to derive a blinking pattern of the user as a reference, such as a camera of the smartphone or a web-camera on the computer. The monitoring unit can be further adapted to access a historical calibration database comprising data from the user or other users. The monitoring unit can further carry out calibration processes. The monitoring unit can be adapted to synchronize the derived/historical/calibration data with a central database. The monitoring unit may be further adapted to be dependent on a user's input complying with the derived data to be shared. When the derived data is to be shared, other users can be able to use the derived data for their own use.

The storage unit may be in the form of a cloud, internet server, mobile phone storage etc.

The at least one eye sensor is an optical sensor which itself can be or includes a light detector. The at least one eye sensor may be referred to as at least one eye activity sensor. The at least one eye sensor may be arranged on glasses of the wearable device, such that a light reflected from a light source can be optimally received. The at least one eye sensor may be in the form of a single point photodetector (i.e. Photodiode or phototransistor), multiple spatially separated point detector (i.e. row of photodiodes) and/or detector array (i.e. point detectors arranged in a grid or a camera). The camera can be of a CCD or CMOS type.

The recorded data can comprise stored data related to the eye activity of the user. The recorded data can further comprise stored data related to the eye activity of other users. The recorded data can further comprise stored data related to the health condition of the user. The recorded data can further comprise stored data related to an input of the user. The recorded data can further comprise stored data related to a health condition or an input of the other users.

The recorded data can be previously stored data. The recorded data can be historical data. The recorded data can indicate the health condition of the user. The recorded data can indicate a health condition of another user or other users.

The recorded data provide the advantage of enabling a better medical treatment of a patient being provided with such a system.

The optical signal can originate from a light source. The light source is an artificial light source. The artificial light source can be mountable on the wearable device.

The system comprises at least one light source. The at least one light source can be adapted to transmit the optical signal to an eye and/or surrounding tissues of the user. The at least one light source can be arranged to transmit the optical signal to an eye and/or surrounding tissues of the user. The at least one light source can further be calibrated. The at least one light source can further be mountable on the wearable device to transmit the optical signal to the eye and/or surrounding tissues of the user. The at least one eye sensor can further be calibrated to be in alignment with the light source for the monitoring unit to optimally derive data related to the eye activity of the user.

The system comprises at least one light detector. The at least one light detector can be adapted to receive the optical signal from an eye and/or surrounding tissues of the user. The at least one light detector can be arranged to receive the optical signal from an eye of the user. The at least one light detector can further be calibrated. The at least one light detector can further be mountable on the wearable device to receive the optical signal reflected from the eye structures, eyelid and/or other eye surrounding tissues of the user. The at least one light detector can further be calibrated to be in alignment with the at least one light source for the monitoring unit to optimally derive data related to the eye activity of the user based on the received optical signal.

A combination of a light source/ambient light with a light detector has the advantage of sensitize the deriving of the data.

The light source can be a single light source, or multiple light sources. The light technology underlying the light source may be a light emitting diode (LED), a superluminiscent diode (SLD), a laser diode and/or specifically directed waveguides to define a path of light. The light source may be further arranged on glasses of the wearable device, such that they are optimally aligned for improving processing results. The light source may be further arranged externally from the wearable device, such as on a desk, computer screen or a mobile device (i.e. smartphone or table).

The eye sensor can be able to be calibrated to a personal condition of the user. The personal condition can comprise an eye size of the user. The personal condition can comprise a relative position of the frame to a position of the eyes of the user. The personal condition can comprise a relative position of the wearable device to the position of the eyes of the user.

A calibration can have the advantage of making the system adaptable to a special user and making the derived data more precise and comparable to other user's extracted data, such as the recorded data.

The monitoring unit may be further adapted to relate (raw) optical signals to an eye activity of the user using the calibration data. The optical signals may be reflections from eye structures, an eyelid and/or other surrounding tissues of the user.

The term "calibration data" may be understood as a data used by an algorithm to relate measured raw signals, such as the derived signal, to the actual eye activity. Calibration data can be in the form of raw measurement data, reference data or context data. The sensor unit and the monitoring unit are adapted to calibrate raw signals (raw signals may comprise context, reference and optical data) by obtaining information about a blink state from an independent reference signal. Such a reference signal can be a direct user input provided through the glasses of the wearable device, like pressing a button on the frame of the wearable device, tapping on the frame detected by an accelerometer on the frame, a head or hand gesture. This feature can be further implemented in a smartphone or mobile device app. For example, the reference signal for the blink detection can be blink detection vision algorithm analysing images from the smartphone camera imaging user's eyes.

The sensor unit may further comprise a context sensor. The context sensor can be adapted to detect another signal related to an environment of the user. The context sensor can be arranged in the sensor unit. The context sensor can be arranged in the monitoring unit. The monitoring unit can further be adapted to derive environmental data included in the other signal by processing the other signal. The storage unit can further be adapted to store the derived environmental data. The monitoring unit can further be adapted to assess the health condition of the user by comparing the recorded data with the derived data related to the eye activity of the user and the derived environmental data. The context sensor may be arranged on the sensor unit and/or the monitoring unit. The context sensor may be a physiological sensor or ambient/environmental sensor. The sensor unit may further comprise an accelerometer/magnetometer/gyroscope, environmental sensor and/or an additional physiological sensor, or a plurality thereof. The accelerometer/magnetometer/gyroscope may be adapted to detect an orientation of the head of the user. The accelerometer/magnetometer/gyroscope may be adapted to detect user activity, such as walking, running, reading, talking, etc. The accelerometer/magnetometer/gyroscope may be adapted to detect a user input, such as tapping, shaking, gestures, etc. The physiological sensor may be adapted to measure vital signs, such as a heart rate, blood oxygenation and/or electro dermal activity, etc. The ambient/environmental sensor may be in the form of a proximity sensor, for example on a frame of the wearable device. The proximity sensor may be directed in a direction of sight and may detect distances to objects. The ambient/environmental sensor may be in the form of and ambient light sensor adapted to measure intensity and/or spectral content of visible and/or infrared and/or ultraviolet ranges, wherein the monitoring unit is further adapted to calculate a UV index to relate it to the eye activity. The ambient/environmental sensor may be in the form of a temperature sensor adapted to measure a temperature, wherein the monitoring unit is adapted to relate the temperature to an activity of the user. The ambient/environmental sensor may be in the form of a humidity sensor adapted to measure a humidity, wherein the monitoring unit is adapted to relate the humidity to an activity of the user. The ambient/environmental sensor may be in the form of a pressure sensor adapted to measure a pressure, wherein the monitoring unit is adapted to relate the pressure to an activity of the user. The ambient/environmental sensor may be in the form of an environmental pollution monitoring unit adapted to determine an amount of pollution in the air, wherein the monitoring unit is further adapted to derive data related to the eye activity, such as blinking triggers based on the pollution. The ambient/environmental sensor may be in the form of a location based sensor adapted to determine the user's location in non-shadowed environments, wherein the monitoring unit is further adapted to calculate a position based on the communication system or an internet connection. The ambient/environmental sensor may be in the form of a camera. The camera may be adapted to e.g. periodically capture images of an environment of the user. The camera can be triggered by the user. The monitoring unit can be further adapted to derive data related to the context comprising information about the environment.

An advantage of the context sensor is to build in environmental and ambient influences in the data to be derived and the stored/recorded data which makes it more flexible in processing, such that further medical issues can be observed and treated. It further makes meaningful physiological interpretation possible.

The term "environmental data" may be understood as context data, which is information about the user. Context data can be information not directly derived from the eye, such as heart rate, perspiration, head orientation, age, etc. Context data can be information about the user's environment, such as temperature, humidity or position. Context data can further be information from a user, provided via text, voice and/or image input, about information, such as being itchy, tired and/or sleepy.

The system can further comprise a user interface. The user interface can be adapted to receive an input from the user. The user can indicate whether the health condition corresponds to the derived eye activity data and/or the derived environmental data. The indication can be performed by an input of the user via the input interface.

The user interface can have the advantage of weighting a determined result of the health condition, such that a health condition can be weighted less when it is determined to be false and it can be weighted less when it is determined right. This as a result can then be stored as recorded data with a specified weighting according to the user's input.

The recorded data can further comprise previously stored calibration data and previously stored environmental data from the user and/or other users.

Further, the monitoring unit and the sensor unit can be adapted to receive calibration data by a calibration process performed by the user. The calibration data and the previously stored calibration data can be data which is used by an algorithm to convert a raw signal from a detector, such as the light detector or the sensor unit, and context data to a physiological parameter of interest. At least part of the calibration data and previously stored calibration data may be in a form of parameters for the algorithm for such a conversion. Further, at least part of the calibration data or previously stored calibration data may be in a form of computer instructions for such a conversion.

The user interface can be further adapted to indicate whether a physical and/or psychological abnormality occurred based on the comparing.

The monitoring unit can further comprise an alarm unit. The alarm unit can be adapted to indicate to a user that a physical and/or psychological abnormality occurred based on the comparing. The indication may be in the form of images, for example projected on the glasses of the wearable device or a vibration alarm or just visible light. Further the indication can be dependent on physiological status of the user, such as informing the user to blink, to relax the eyes and/or to reduce an exposure to harmful environment, and/or dependent on a status of the monitoring unit or sensor unit, such as a battery status, connection status etc. The indication can further be dependent on external conditions or events, such as an incoming phone call, drop of outside temperature etc. The alarm unit may further be adapted to be in the form of an input unit and to receive a user input via a button/accelerometer based tap detector. The monitor unit may further be adapted based on an indication from the alarm unit to display information to the user and/or alarm the user.

The alarm unit can have the advantage of providing a user/patient with direct information, such that the user/patient can take quick responsibility in a medical or treatment scenario where fast reaction is required.

The system comprises an additional sensor, which is a smartphone camera. A front and/or back camera of the smartphone can be used. The additional sensor is adapted to obtain another optical signal reflected from the eye of the user. The monitoring unit, for example the smartphone, can be adapted to perform an image analysis algorithm. The image analysis algorithm is adapted to identify blinks of the user and to relate the identified blinks of the user to the data related to the eye activity.

According to a second aspect, not part of the claimed subject-matter, a method for assessing a health condition of a user comprises obtaining, by a sensor unit, an optical signal reflected from an eye of the user. The sensor unit can be mounted on a wearable device. The method further comprises deriving, by a monitoring unit connected to the sensor unit, data related to an eye activity of the user by processing the optical signal. The data related to the eye activity of the user is included in the optical signal. The method further comprises storing, by a storage unit connected to the monitoring unit, the derived data related to the eye activity of the user and recorded data. The method further comprises obtaining, by the monitoring unit, the recorded data from the storage unit. The method further comprises assessing, by the monitoring unit, the health condition of the user by comparing the recorded data with the derived data related to the eye activity of the user.

Even if some of the aspects described above have been described in reference to the system, these aspects may also apply to the method. Likewise, the aspects described above in relation to the method may be applicable in a corresponding manner to the system.

Other objects, features, advantages and applications will become apparent from the following description of non-limiting embodiments with reference to the accompanying drawings. In the drawings, all described and/or illustrated features, alone or in any combination form the subject matter disclosed therein, irrespective of their grouping in the claims or their relations/references. The dimensions and proportions of components or parts shown in the figures are not necessarily to scale; these dimensions and proportions may differ from illustrations in the figures and implemented embodiments.

The figures show:
- Fig. 1: schematically illustrates a system implementation according to an embodiment;
- Fig. 2: schematically illustrates a method implementation according to an embodiment;
- Fig. 3: schematically illustrates a flow chart according to a method implementation of an embodiment;
- Fig. 4: schematically illustrates an exemplary system implementation according to an embodiment;
- Fig. 5: schematically illustrates a light detector and light source implementation on a wearable device according to an embodiment;
- Fig. 6: schematically illustrates a flow diagram exemplifying an algorithmic process according to an embodiment; and
- Fig. 7: schematically illustrates a diagram representing a blinking signal comprised in an optical signal according to an embodiment.

The variants of the functional and operational aspects as well as their functional and operational aspects described herein are only for a better understanding of its structure, its functions and properties; they do not limit the disclosure to the embodiments. The figures are partially schematic, said essential properties and effects are clearly shown enlarged in part in order to clarify the functions, active principles, embodiments and technical characteristics. Every operation, every principle, every technical aspect and every feature that/which is disclosed in the figures or in the text is/are able to be combined with all claims, each feature in the text and the other figures, other modes of operation, principles, technical refinements and features that are included in this disclosure, or result from it, so that all possible combinations are assigned to the devices and methods described. They also include combinations of all individual comments in the text, that is, in each section of the description, in the claims and combinations between different variations in the text, in the claims and in the figures, and can be made to subject-matter of further claims. The claims do not limit the disclosure and therefore the possible combinations of all identified characteristics among themselves. All features disclosed are explicitly also individually and in combination with all other features disclosed herein.

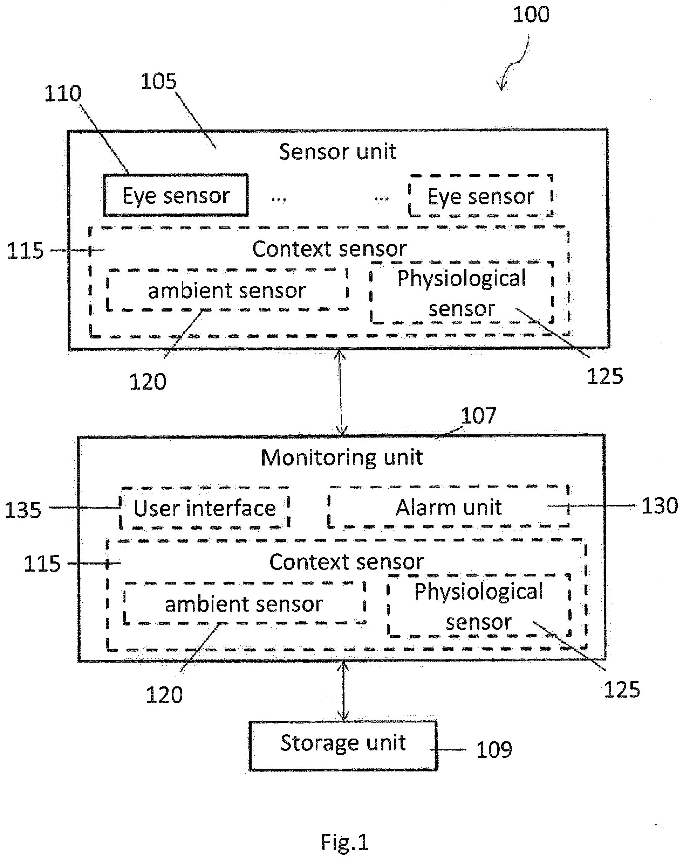

Figure 1 schematically illustrates a system 100 implementation according to an embodiment of the invention. The system 100 for assessing a health condition of a user comprises a sensor unit 105, a monitoring unit 107 and a storage unit 109. The sensor unit 105 comprises at least one eye sensor 110. The at least one eye sensor 110 is adapted to obtain an optical signal reflected from an eye of the user. The optical signal can be generated by an artificial light or can be gathered by the at least one eye sensor 110 using an ambient light reflected by at least one of the user's eyes. The artificial light can be connected to the sensor unit 105 or the monitoring unit 107. The sensor unit 105 can be mounted on a wearable device. The wearable device can be adapted to mount the sensor unit 105, the monitoring unit 107, the storage unit 109 and/or the artificial light/light source. The monitoring unit 107 is connected to the sensor unit 105. This connection can be established via a Bluetooth connection. The monitoring unit 107 is adapted to derive data related to an eye activity of the user by processing the optical signal. The data related to the eye activity of the user is included in the optical signal. The storage unit 109 is connected to the monitoring unit 107. This connection may be established via an internet connection. The storage unit 109 is adapted to store the derived data related to the eye activity of the user and recorded data. The monitoring unit 107 is further adapted to obtain the recorded data from the storage unit 109. The monitoring unit 107 is further adapted to assess the health condition of the user by comparing the recorded data with the derived data related to the eye activity of the user.

Data related to the eye activity of the user can be data related to blinking and eye movements, for example to assess dry eye monitoring. It is an advantage of the present invention to be able to extract parameters of interest from the data related to the eye activity. For example, the storage unit 109 can be a database of calibration data available from other users. Further this calibration data can be recorded data from the user who is currently wearing the wearable device. Further, it is advantages to use context data, such as environmental and physiology data to further improve an accuracy of the data related to the eye activity. Further, the context data can enhance the data for more meaningful physiologic interpretation. In figure 1, the context data is collected by the context sensor 115. The context sensor 115 can comprise an ambient sensor 120 and/or a physiological sensor 125. The ambient sensor 120 can be adapted and arranged to collect data from the surrounding environment. The physiological sensor 125 can be adapted and arranged to collect data related to human vitals of the user. Further, the sensor unit and the monitoring unit can be separate units or comprised in the same unit. The monitoring unit 107 can comprise a user interface 135, an alarm unit 130 and the context sensor 115. The user interface 135 can be adapted and arranged to receive an user input from the user wearing the wearable device, such as the spectacles mounting the sensor unit. Further, the user interface 135 can be adapted to interact with the monitoring unit, such that a user is able to weigh an importance of gathered/collected data related to the eye activity. The alarm unit 130 may be adapted and arranged to alarm/signal to the user that something related to the eye activity or the environment or his/her physiology may need to be adjusted. The monitoring unit 107 can be connected to the storage unit 109, in such a way that the monitoring unit 107 can derive data, which might be recorded data/stored data or currently processed data from the storage unit 109 in order to compare it with the currently processed/derived data. The storage unit 109 can be a server/database, cloud or any kind of storage which is able to be accessed over the Internet.

As for example, one can consider blinking activity and a physiological dry eye condition as a medical problem. The eye sensor mountable on a frame of a pair of spectacles gathers eye activity data providing after a calibration a signal of blinking events. For example, a user exhibits specific blinking statistics such as frequency in the simplest case. Then a product specific application (app) on a smartphone can automatically query a cloud database and takes into account other parameters, such as user's activity or ambient humidity, which may also be monitored by another sensor such as a context sensor 115 mounted on the wearable device. The results could indicate that a majority of users with such a blinking pattern and under those specific ambient conditions later reported all were diagnosed with an onset of the dry eye problem. The system can be used so that the user can be advised to take specific actions. Predictive analytics can be performed on the cloud based on historical data to warn the user in advance. In an even simpler case, an algorithm can predict that a long period of non-blinking through the day might result in itchiness and redness of the eyes in the evening, for example while watching TV or working on the PC which might be self-reported or detected by reference sensors such as a smartphone camera. It is an advantage of the present invention to use historical data and relate to blinking to the user's health condition. This is also achieved by the concept of connecting the device to access the cloud data analytics which allows to solve the problem of a device calibration. The device can be understood as the sensor unit 105. Further, automatic calibration of the device with reference states such as eyes closed or open, looking to the left or right, up or down, etc. based on a reference method for example camera of the smartphone to which the device is connected. The simple use case scenario is that the user puts the wearable device on, for example glasses, starts a control application on the monitoring unit, which activates the camera of the smartphone for monitoring an eye activity. Then the user is to open/close the eyes which is automatically detected by the application capturing images from the smartphone camera and related to the signals measured by the sensor unit/eye sensor mounted on the wearable device. After calibration has completed, the smartphone application deactivates the camera and the user can proceed with his or her normal daily activities, while the sensor unit 105 in connection with the monitoring unit 107 is enabled to detect a blinking pattern of the user. Further, the calibration of raw signals gathered by the sensor unit 105 is based on the historical/recorded data available for the same user of other users in combination with the context data such as age, sex, facial features, etc. The system 100 enables the user to infer physiological and/or psychological information based on the historical/recorded data from the same or other users and related to the self-reported status. The physiological data can be an onset of dry eye disease. The psychological data can be stress, drowsiness, etc.

Eye activity can be understood as an adjustment of the eyes' psychological state. This includes blinks, eye movements including rotation, pupil size changes, accommodation, etc. Context data can be information about the user, which is not directly derived from the eye (heart rate, perspiration, head orientation, age, etc.), the user's environment (temperature, humidity, location) and text fields, voice input or images to add information from the user himself/herself (i.e. itchy, tired, sleepy). Calibration data can be the data which is used by an algorithm to convert raw signals from the sensor unit and context data to the physiological parameters of interest. At least part of the calibration data may be in a form of parameters for the algorithm for such conversions. At least part of the calibration data may be in a form of computer instructions for such conversions. The sensor unit 105 and the monitoring unit 107 may be able to detect and relate raw sensor measurement signals like light reflections from the eye to the actual eye activity using calibration data. The derived eye activity parameters of interest might be a frequency of blinks, such as blinks per minute, interblink interval in seconds, eye movements in vertical and horizontal direction, pupil radius, blink completeness and other such as refraction. The purpose of the sensor unit 105 is to obtain raw signals from the eye and the relevant context data. The sensor unit 105 may be mounted on a spectacles frame. This spectacles frame can be a dedicated frame or normal glasses suitable to hold the sensor unit 105. The monitoring unit 107 can be a separate device also mounted on the spectacles frame. The monitoring unit 107 can carry out the processing of the raw data, evaluating the parameters and controlling and exchanging of data between the sensor unit 105 and the storage unit 109, which can be a network storage (cloud).

The sensor unit 105 may be implemented in a glasses frame with an attached proximity sensor and an infrared LED located in front of one or both of the user's eyes. The proximity sensor may be connected to a microcontroller board (which may also be attached on the glasses' frame such as the spectacles temple) via a digital bus. The proximity sensor can also act as an ambient light sensor. The sensor modulates the intensity of LED and subtracts ambient background. It delivers a signal related to the proximity of an object in front of the user. Closer (or more reflective) objects lead to a higher signal level. The microcontroller board may have a battery as a power source, micro USB interface for charging and a 3-D accelerometer and temperature sensor. The board can communicate with the monitoring unit 107, such as a smartphone or tablet, via a low-energy Bluetooth interface. The smartphone/tablet may run an app which reads out the measurements of the proximity sensor, accelerometer and temperature sensor and may further be adapted to store them into a data file for post-processing. The smartphone/tablet may perform an analysis of the signal and to identify blinks by predefined signatures of a signal change. This is then shown as statistics of the blinks to the user. Further, the at least one eye sensor 110 may contain multiple sources and/or detectors in order to be able to identify and use optimal combinations/mixtures of signals for an individual or for a current fitting of the glasses/spectacles.

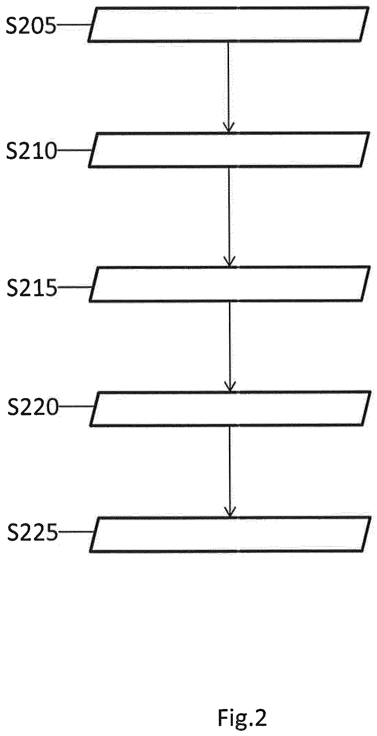

To understand the principle of the present invention, figure 2 schematically illustrates a method implementation according to an embodiment.

Figure 2 schematically shows the method according to an embodiment of the invention for assessing a health condition of a user. The method comprises obtaining S205, by a sensor unit, an optical signal reflected from an eye of the user. The sensor unit can be mounted on a wearable device. The method further comprises deriving S210, by a monitoring unit connected to the sensor unit, data related to an eye activity of the user by processing the optical signal. The data related to the eye activity of the user is included in the optical signal. The method further comprises storing S215, by a storage unit connected to the monitoring unit, the derived data related to the eye activity of the user and recorded data. The method further comprises obtaining S220, by the monitoring unit, the recorded data from the storage unit. The method further comprises assessing S225, by the monitoring unit, the health condition of the user by comparing the recorded data with the derived data related to the eye activity of the user.

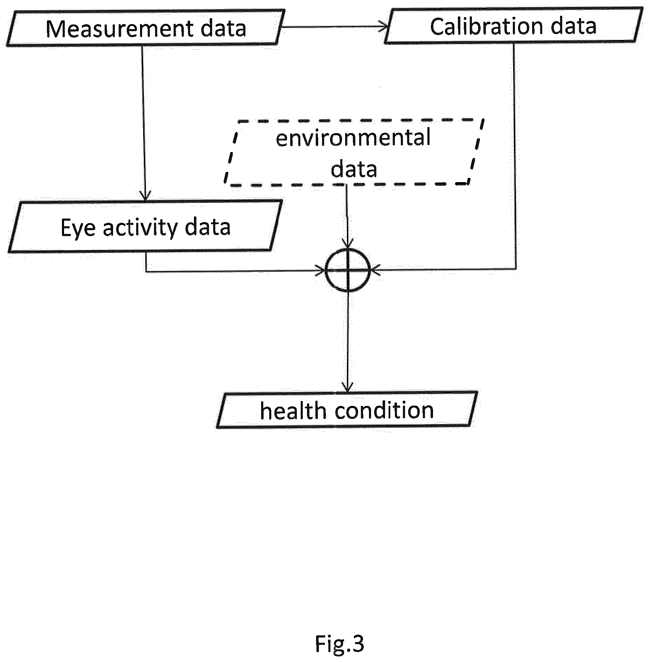

For a better understanding, another embodiment of the method for assessing a health condition of a user is shown in figure 3.

Figure 3 schematically illustrates a flow chart according to a method implementation of an embodiment of the invention. In figure 3, measurement data, calibration data, eye activity data and environmental data are illustrated to be combined in order to extract a health condition. The measurement data can be data related to an eye activity currently measured by the sensor unit/eye sensor according to figure 1. The measurement data can be calibration data obtained by a calibration technique performed by the user before using the wearable device comprising the sensor unit. Further, the calibration data can be data from the same or other users obtained in earlier sessions of using the wearable device. The wearable device can be a frame/spectacles. In order to obtain calibration data, the user/other users need to wear the wearable device in order to gather calibration data/historical data. The historical data, herein also referred to as recorded/stored data, can be used in a step of comparison with the currently derived data while the user uses the wearable device to obtain data. Further, environmental data can be gathered in conjunction with the calibration data and the eye activity data in order to more accurately derive and/or assess a health condition of the user.

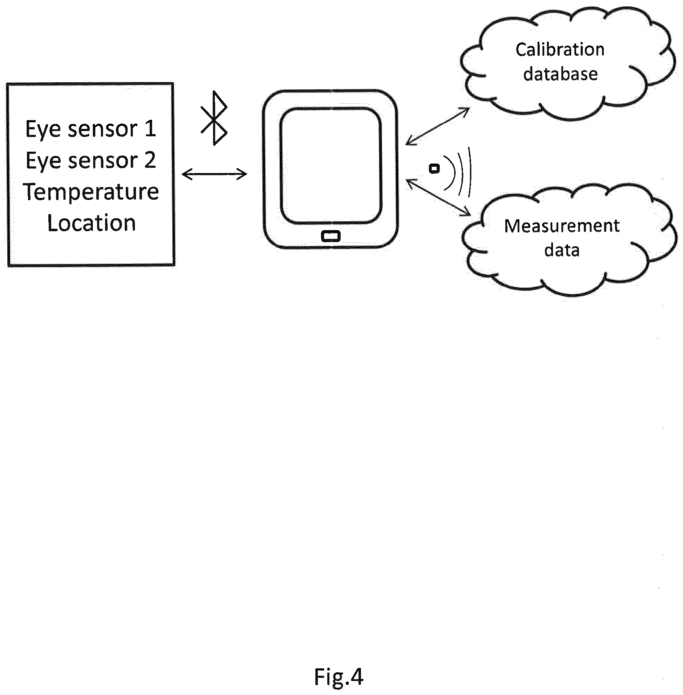

In order to ease the understanding of the method implementation according to the foregoing disclosure, an exemplary system implementation is shown in figure 4.

Figure 4 schematically illustrates an exemplary system implementation according to an embodiment of the invention. Different kinds of sensors embodied as the sensor unit according to figure 1 are illustrated on the left-hand side of the figure, such as eye sensor 1 and the eye sensor 2, and a temperature and location sensor. This is just an exemplary sensor arrangement, such that it can also only comprise one eye sensor and one of the temperature and the location sensors for example. The sensor unit is for example connected to the monitoring unit, here illustrated as a smartphone device, via a Bluetooth connection. The monitoring unit itself is for example connected to a calibration database and a measurement database via wireless communication. For example, the bluetooth connection and the wireless communication connection can be switched on and off via a user interface by the user. This enables the monitoring unit to extract data from the calibration database in combination with the measurement data. The measurement data can be the derived data, and the calibration data from the calibration database can be the recorded data. The monitoring unit 107 may comprise another type of sensor, such as a location-based sensor for example GPS, GLONASS or GNSS sensors. In some situations in which it is difficult to know a user's location, the user may aid in the determination of his or her location and or context information via an input. The user input can be processed by the user interface, for example comprised in the monitoring unit. If the location sensor provides data making it difficult to determine if a user is for example in a car or a bus, the monitoring unit and a cloud server communicating with the monitoring unit may present a query to the user asking if he or she took the bus or the car. Similar queries may occur for locations other than vehicular contexts. For example, if the data related to the eye activity, the user's psychological or physiological state and/or the environment indicates that the user completed a specific task, such as a vigorous workout, but there is no location data that indicates that the user went to a gym, the user may be asked if he/she were to the gym today.

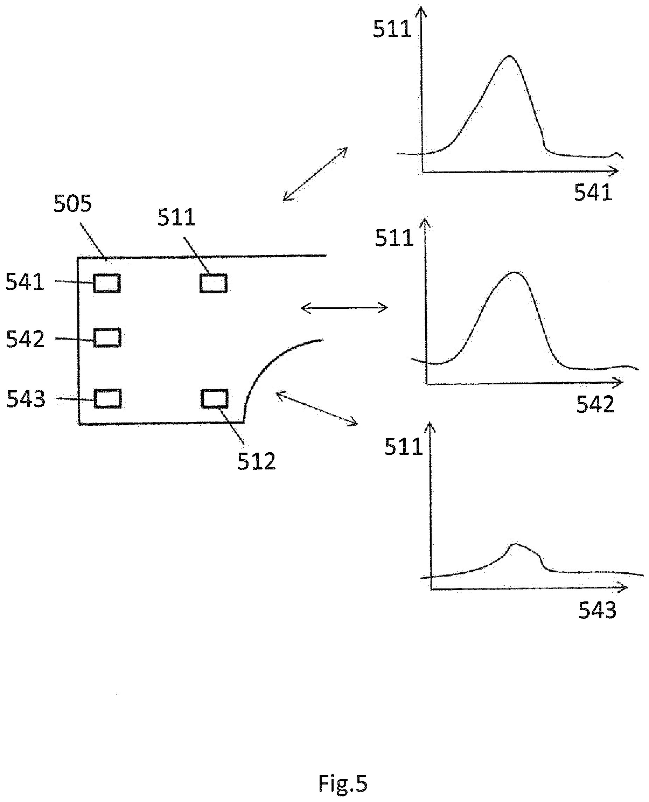

In order to illustrate how data is derived by a wearable device according to an embodiment of the invention, figure 5 illustrates an exemplary detector/source arrangement with corresponding diagrams comprising resulting data.

Figure 5 schematically illustrates a light detector/sensor and light source implementation on a wearable device according to an embodiment of the invention. The wearable device 505 can comprise light sources 541, 542 and 543. The wearable device can further comprise light detectors 511 and 512. On the right-hand side of figure 5, diagrams for the relation between the three different light sources 541, 542 and 543 with the light detector 511 are illustrated. The shortest distance between the light source and the light detector happens to lead to the highest amount of output data, thereby leading to a higher peak in the related diagram. The lowest amount of output data happens to correspond to the longest distance between the light source and the light detector, as shown in figure 5. The eyes of different users can be located in different positions in relation to the frame of the wearable device 505, light source and light detector. For example, for a user with eyes located closer to the top of the frame of the wearable device 505, the light detector may receive a better signal from a combination of the light source 541 and the light detector 511. For a user with eyes closer to the bottom of the frame, a combination of the light source 543 and the light detector 512 may be preferred.

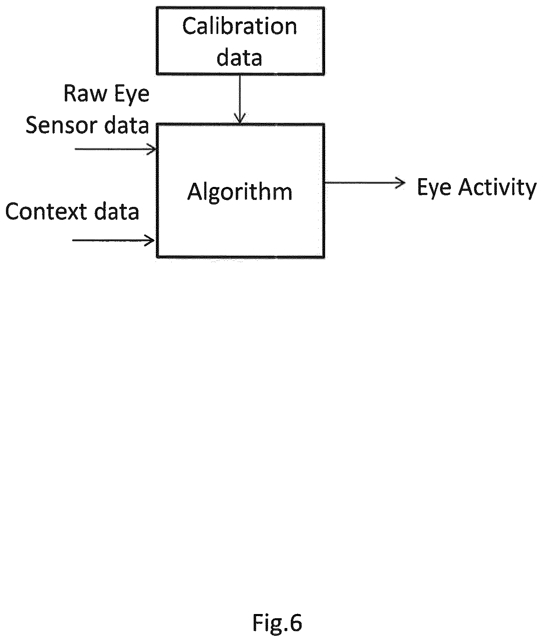

In order to illustrate how the resulting data can be used in obtaining an eye activity, figure 6 schematically illustrates the use of an algorithm for obtaining an eye activity by combining context data and raw eye sensor data, which is also referred to as the optical signal in this disclosure. The raw eye sensor data can be the detected data by the eye sensor.

Figure 6 schematically illustrates a flow diagram exemplifying an algorithmic process according to an embodiment of the invention. Raw eye sensor data and context data can be gathered and used as input data for the algorithm. Further, calibration data can be used as another input data for the algorithm. Calibration data may be understood as data used by the algorithm to relate the measured raw signals to the actual eye activity. This may be performed by a mapping algorithm. The calibration data may consist of raw measurement data, reference data and context data. The system may be able to calibrate the signal by obtaining information about the blink state from an independent reference signal. Such reference signal may be a direct user input provided through the user interface. The user interface may be arranged on the wearable device. The user input may be represented by pressing a button on the frame of the wearable device, tapping on the frame (for example single tap means eyes open, double tap means eyes closed) detected by an accelerometer being arranged on the frame of the wearable device or a head gesture (for example nodding indicates blink, shaking head indicates eyes open). The user input can be given on a controller with means of pressing a button, tapping if the controller hardware/software allows this kind of operation. This may also be implemented in an app by clicking, or shaking for example the smartphone. When the algorithm is performed, data related to the eye activity is extracted by a combination of the data, such as it is done in figure 3.

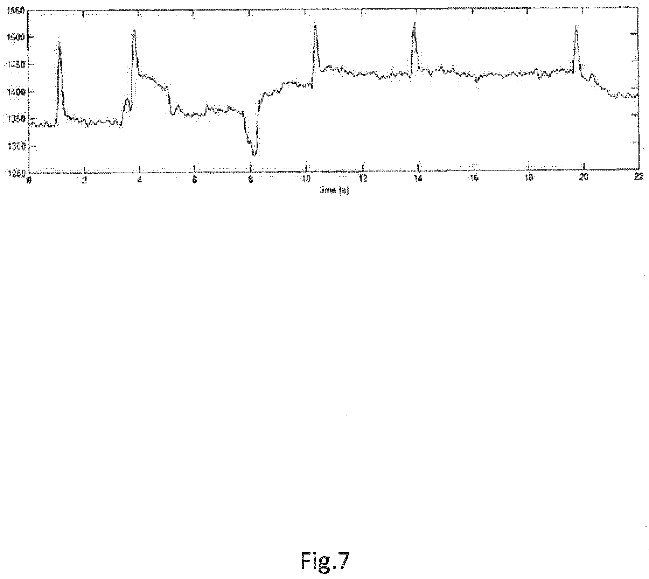

In order to illustrate the data related to an eye activity, figure 7 illustrates a timely variation of the data gathered by an eye sensor.

Figure 7 schematically illustrates a diagram representing a blinking signal comprised in an optical signal according to an embodiment of the invention. This data is extracted from a sensor output showing an illustration of a blinking signal as detected from an eye reflection.

In order to obtain a reference signal, the system according to an embodiment of the present invention can comprise an additional sensor. For example the additional sensor can be comprised in the monitoring unit. For example, if the monitoring unit is a smartphone, the front/back camera can be used and an image analysis algorithm can be adapted to identify blinks of the user and relate them to a primary blink signal without a direct user input via the user interface. This may be referred to as passive monitoring, wherein the reference data analysis can be performed in real-time and/or retrospectively. The retrospective analysis can benefit from a direct user input in a manual or semi-automatic mode. For example in the manual mode, the user is shown the images and is asked to judge the status of the eye (for example blink or open eye). In the semi-automatic mode, the algorithm is identifying the eye status and only shows the user the result with the reference data and asks to confirm or reject the result (for example left or right swipe on the smartphone screen to accept or reject results respectively). As the calibration data can comprise context data, the calibration data can be related to additional physiological or ambient data to improve the accuracy of the eye activity detection. Context data can have a form of static information, like age, body weight, skin type, eyes colour, information about health, etc., an image of the face or in a form of monitored parameters like a user's movement, ambient temperature, etc. Context data can also be used in relation with the eye activity data in order to derive context related statistics.

## Claims

1. A system (100) for assessing a health condition of a user comprising:
at least one light source adapted to transmit a first optical signal to an eye of the user, the light source being mountable on a wearable device;
a sensor unit (105) comprising at least one eye sensor (110) adapted to obtain a second optical signal reflected from the eye of the user, wherein the sensor unit (105) can be mounted on the wearable device;
a monitoring unit (107) connected to the sensor unit (105) and adapted to derive data related to an eye activity of the user by processing the second optical signal;
a storage unit (109) connected to the monitoring unit (107) and adapted to store the derived data related to the eye activity of the user and recorded data; and
wherein the monitoring unit (107) is further adapted to obtain the recorded data from the storage unit (109) and to assess the health condition of the user by comparing the recorded data with the derived data related to the eye activity of the user, and
wherein the system (100) further comprises an additional sensor adapted to obtain a third optical signal reflected from the eye of the user, wherein the additional sensor is a camera of a smartphone, and the third optical signal is images captured by the camera,
**characterized in that**:
the monitoring unit (107) is adapted to perform an image analysis algorithm, which is adapted to identify blinks of the user by using the captured images and is adapted to relate the identified blinks of the user to the data related to the eye activity derived by processing the second optical signal.

2. The system (100) according to claim 1, wherein the recorded data comprises stored data related to the eye activity of the user, stored data related to the eye activity of other users, stored data related to the health condition of the user and/or stored data related to a health condition of the other users.

3. The system (100) according to any one of the foregoing claims, wherein the recorded data is previously stored data and/or historical data, which indicates the health condition of the user and/or a health condition of another user or other users.

4. The system (100) according to any one of the foregoing claims, wherein the light source is an ambient light and/or an artificial light source, the artificial light source being mountable on the wearable device.

5. The system (100) according to any one of the foregoing claims,
wherein the at least one light source can further be calibrated to transmit the first optical signal to the eye and/or surrounding tissues of the user; and
wherein the at least one eye sensor (110) can further be calibrated to be in alignment with the light source for the monitoring unit (107) to optimally derive data related to the eye activity of the user.

6. The system (100) according to claim 5, wherein the at least one eye sensor can further be calibrated and is mountable on the wearable device to receive the second optical signal from the eye and/or surrounding tissues of the user; and
wherein the at least one eye sensor can further be calibrated to be in alignment with the at least one light source for the monitoring unit (107) to optimally derive data related to the eye activity of the user based on the received second optical signal.

7. The system (100) according to any one of the foregoing claims, wherein the eye sensor (110) is able to be calibrated to a personal condition of the user comprising an eye size of the user and/or a relative position of a frame or the wearable device to a position of the eyes of the user.

8. The system (100) according to any one of the foregoing claims, the sensor unit (105) further comprising:
a context sensor (115) adapted to detect a signal related to an environment of the user, wherein the context sensor (115) is arranged in the sensor unit (105) or the monitoring unit (107);
wherein the monitoring unit (107) is further adapted to derive environmental data by processing the signal;
wherein the storage unit (109) is further adapted to store the derived environmental data; and wherein the monitoring unit (107) is further adapted to assess the health condition of the user by comparing the recorded data with the derived data related to the eye activity of the user and the derived environmental data.

9. The system (100) according to any one of the foregoing claims, further comprising:
a user interface (135) adapted to receive an input from the user, wherein the user indicates whether the health condition corresponds to the derived eye activity data and/or the derived environmental data.

10. The system (100) according to claim 9, wherein the user interface (135) is further adapted to indicate whether a physical and/or psychological abnormality occurred based on the comparing.

11. The system (100) according to any one of the foregoing claims, wherein the recorded data comprises previously stored calibration data and previously stored environmental data from the user and/or other users.

12. The system (100) according to any one of the foregoing claims, wherein the monitoring unit further comprises:
an alarm unit (130) adapted to indicate to a user that a physical and/or psychological abnormality occurred based on the comparing.

## Patentansprüche

1. System (100) zum Beurteilen eines Gesundheitszustands eines Benutzers, das Folgendes umfasst:
mindestens eine Lichtquelle, die dazu eingerichtet ist, ein erstes optisches Signal an ein Auge des Benutzers zu übertragen, wobei die Lichtquelle an einer tragbaren Vorrichtung montierbar ist;
eine Sensoreinheit (105), die mindestens einen Augensensor (110) umfasst, der dazu eingerichtet ist, ein zweites optisches Signal zu erhalten, das von dem Auge des Benutzers reflektiert wird, wobei die Sensoreinheit (105) an der tragbaren Vorrichtung montiert werden kann;
eine Überwachungseinheit (107), die mit der Sensoreinheit (105) verbunden ist und dazu eingerichtet ist, Daten abzuleiten, die mit einer Augenaktivität des Benutzers in Beziehung stehen, indem das zweite optische Signale verarbeitet wird;
eine Speichereinheit (109), die mit der Überwachungseinheit (107) verbunden ist und dazu eingerichtet ist, die abgeleiteten Daten, die mit der Augenaktivität des Benutzers in Beziehung stehen, und aufgezeichnete Daten zu speichern; und
wobei die Überwachungseinheit (107) ferner dazu eingerichtet ist, die aufgezeichneten Daten von der Speichereinheit (109) zu erhalten und den Gesundheitszustand des Benutzers zu beurteilen, indem die aufgezeichneten Daten mit den abgeleiteten Daten, die mit der Augenaktivität des Benutzers in Beziehung stehen, verglichen werden, und
wobei das System (100) ferner einen zusätzlichen Sensor umfasst, der dazu eingerichtet ist, ein drittes optisches Signal zu erhalten, das von dem Auge des Benutzers reflektiert wird, wobei der zusätzliche Sensor eine Kamera eines Smartphones ist, und das dritte optische Signal Bilder ist, die durch die Kamera aufgenommen werden,
**dadurch gekennzeichnet, dass**:
die Überwachungseinheit (107) dazu eingerichtet ist, einen Bildanalysealgorithmus durchzuführen, der dazu eingerichtet ist, Blinzelvorgänge des Benutzers unter Verwendung der aufgenommenen Bilder zu identifizieren, und dazu eingerichtet ist, die identifizierten Blinzelvorgänge des Benutzers mit den Daten, die mit der Augenaktivität in Beziehung stehen, die durch Verarbeiten des zweiten optischen Signals abgeleitet wird, in Beziehung zu setzen.

2. System (100) nach Anspruch 1, wobei die aufgezeichneten Daten gespeicherte Daten, die sich auf die Augenaktivität des Benutzers beziehen, gespeicherte Daten, die sich auf die Augenaktivität anderer Benutzer beziehen, gespeicherte Daten, die sich auf den Gesundheitszustand des Benutzers beziehen, und/oder gespeicherte Daten, die sich auf einen Gesundheitszustand der anderen Benutzer beziehen, umfassen.

3. System (100) nach einem der vorhergehenden Ansprüche, wobei die aufgezeichneten Daten zuvor gespeicherte Daten und/oder historische Daten sind, die den Gesundheitszustand des Benutzers und/oder einen Gesundheitszustand eines anderen Benutzers oder anderer Benutzer angeben.

4. System (100) nach einem der vorhergehenden Ansprüche, wobei die Lichtquelle ein Umgebungslicht und/oder eine künstliche Lichtquelle ist, wobei die künstliche Lichtquelle an der tragbaren Vorrichtung montierbar ist.

5. System (100) nach einem der vorhergehenden Ansprüche,
wobei die mindestens eine Lichtquelle ferner kalibriert werden kann, um das erste optische Signal an das Auge
und/oder umgebende Gewebe des Benutzers zu übertragen; und
wobei der mindestens eine Augensensor (110) ferner kalibriert werden kann, um sich in Ausrichtung mit der Lichtquelle für die Überwachungseinheit (107) zu befinden, um Daten, die mit der Augenaktivität des Benutzers in Beziehung stehen, optimal abzuleiten.

6. System (100) nach Anspruch 5,
wobei der mindestens eine Augensensor ferner kalibriert werden kann und an der tragbaren Vorrichtung montiert werden kann, um das zweite optische Signal von dem Auge und/oder umgebenden Geweben des Benutzers zu empfangen; und
wobei der mindestens eine Augensensor ferner kalibriert werden kann, um sich in Ausrichtung mit der mindestens einen Lichtquelle für die Überwachungseinheit (107) zu befinden, um Daten, die mit der Augenaktivität des Benutzers in Beziehung stehen, basierend auf dem empfangenen zweiten optischen Signal optimal abzuleiten.

7. System (100) nach einem der vorhergehenden Ansprüche, wobei der Augensensor (110) in der Lage ist, auf einen persönlichen Zustand des Benutzers kalibriert zu werden, der eine Augengröße des Benutzers und/oder eine relative Position eines Gestells oder der tragbaren Vorrichtung zu einer Position der Augen des Benutzers umfasst.

8. System (100) nach einem der vorhergehenden Ansprüche, wobei die Sensoreinheit (105) ferner umfasst:
einen Kontextsensor (115), der dazu eingerichtet ist, ein Signal zu detektieren, das mit einer Umgebung des Benutzers in Beziehung steht, wobei der Kontextsensor (115) in der Sensoreinheit (105) oder der Überwachungseinheit (107) angeordnet ist;
wobei die Überwachungseinheit (107) ferner dazu eingerichtet ist, Umgebungsdaten durch Verarbeiten des Signals abzuleiten;
wobei die Speichereinheit (109) ferner dazu eingerichtet ist, die abgeleiteten Umgebungsdaten zu speichern; und wobei die Überwachungseinheit (107) ferner dazu eingerichtet ist, den Gesundheitszustand des Benutzers zu beurteilen, indem die aufgezeichneten Daten mit den abgeleiteten Daten, die mit der Augenaktivität des Benutzers in Beziehung stehen, und den abgeleiteten Umgebungsdaten verglichen werden.

9. System (100) nach einem der vorhergehenden Ansprüche, das ferner Folgendes umfasst:
eine Benutzerschnittstelle (135), die dazu eingerichtet ist, eine Eingabe von dem Benutzer zu empfangen, wobei der Benutzer angibt, ob der Gesundheitszustand den abgeleiteten Augenaktivitätsdaten und/oder den abgeleiteten Umgebungsdaten entspricht.

10. System (100) nach Anspruch 9, wobei die Benutzerschnittstelle (135) ferner dazu eingerichtet ist, basierend auf dem Vergleich anzugeben, ob eine physische und/oder psychologische Anomalie aufgetreten ist.

11. System (100) nach einem der vorhergehenden Ansprüche, wobei die aufgezeichneten Daten zuvor gespeicherte Kalibrierungsdaten und zuvor gespeicherte Umgebungsdaten von dem Benutzer und/oder anderen Benutzern umfassen.

12. System (100) nach einem der vorhergehenden Ansprüche, wobei die Überwachungseinheit ferner Folgendes umfasst:
eine Alarmeinheit (130), die dazu eingerichtet ist, einem Benutzer basierend auf dem Vergleich anzugeben, dass eine physische und/oder psychische Anomalie aufgetreten ist.

## Revendications

1. Système (100) permettant d'évaluer un état de santé d'un utilisateur comprenant :
au moins une source de lumière conçue pour transmettre un premier signal optique à un œil de l'utilisateur, la source de lumière pouvant être montée sur un dispositif à porter sur soi ;
une unité de capteur (105) comprenant au moins un capteur oculaire (110) conçu pour obtenir un deuxième signal optique réfléchi par l'œil de l'utilisateur, l'unité de capteur (105) pouvant être montée sur le dispositif à porter sur soi ;
une unité de surveillance (107) connectée à l'unité de capteur (105) et conçue pour dériver des données relatives à une activité oculaire de l'utilisateur en traitant le deuxième signal optique ;
une unité de stockage (109) connectée à l'unité de surveillance (107) et conçue pour stocker les données dérivées relatives à l'activité oculaire de l'utilisateur et les données enregistrées ; et
dans lequel l'unité de surveillance (107) est en outre conçue pour obtenir les données enregistrées en provenance de l'unité de stockage (109) et pour évaluer l'état de santé de l'utilisateur en comparant les données enregistrées aux données dérivées relatives à l'activité oculaire de l'utilisateur, et
dans lequel le système (100) comprend en outre un capteur supplémentaire conçu pour obtenir un troisième signal optique réfléchi par l'œil de l'utilisateur, dans lequel le capteur supplémentaire étant une caméra d'un téléphone intelligent, et le troisième signal optique étant des images capturées par la caméra,
**caractérisé en ce que** :
l'unité de surveillance (107) est conçue pour réaliser un algorithme d'analyse d'image, qui est conçu pour identifier les clignements de l'utilisateur à l'aide des images capturées et est conçu pour relier les clignements identifiés de l'utilisateur aux données relatives à l'activité oculaire dérivée par le traitement du deuxième signal optique.

2. Système (100) selon la revendication 1, dans lequel les données enregistrées comprennent des données stockées relatives à l'activité oculaire de l'utilisateur, des données stockées relatives à l'activité oculaire d'autres utilisateurs, des données stockées relatives à l'état de santé de l'utilisateur et/ou des données stockées relatives à un état de santé des autres utilisateurs.

3. Système (100) selon l'une quelconque des revendications précédentes, dans lequel les données enregistrées sont des données précédemment stockées et/ou des données historiques, qui indiquent l'état de santé de l'utilisateur et/ou un état de santé d'un autre utilisateur ou d'autres utilisateurs.

4. Système (100) selon l'une quelconque des revendications précédentes, dans lequel la source de lumière est une lumière ambiante et/ou une source de lumière artificielle, la source de lumière artificielle pouvant être montée sur le dispositif à porter sur soi.

5. Système (100) selon l'une quelconque des revendications précédentes,
dans lequel l'au moins une source de lumière peut en outre être étalonnée pour transmettre le premier signal optique à l'œil
et/ou aux tissus environnants de l'utilisateur ; et
dans lequel l'au moins un capteur oculaire (110) peut en outre être étalonné pour être aligné sur la source de lumière pour que l'unité de surveillance (107) dérive de manière optimale des données relatives à l'activité oculaire de l'utilisateur.

6. Système (100) selon la revendication 5,
dans lequel l'au moins un capteur oculaire peut en outre être étalonné et peut être monté sur le dispositif à porter sur soi pour recevoir le deuxième signal optique provenant de l'œil et/ou des tissus environnants de l'utilisateur ; et
dans lequel l'au moins un capteur oculaire peut en outre être étalonné pour être aligné sur l'au moins une source de lumière pour que l'unité de surveillance (107) dérive de manière optimale des données relatives à l'activité oculaire de l'utilisateur sur la base du deuxième signal optique reçu.

7. Système (100) selon l'une quelconque des revendications précédentes, dans lequel le capteur oculaire (110) peut être étalonné par rapport à un état personnel de l'utilisateur comprenant une taille d'œil de l'utilisateur et/ou une position relative d'une monture ou du dispositif à porter sur soi par rapport à une position des yeux de l'utilisateur.

8. Système (100) selon l'une quelconque des revendications précédentes, l'unité de capteur (105) comprenant en outre :
un capteur de contexte (115) conçu pour détecter un signal relatif à un environnement de l'utilisateur, dans lequel le capteur de contexte (115) est agencé dans l'unité de capteur (105) ou l'unité de surveillance (107) ;
dans lequel l'unité de surveillance (107) est en outre conçue pour dériver des données environnementales par traitement du signal ;
dans lequel l'unité de stockage (109) est en outre conçue pour stocker les données environnementales dérivées ; et dans lequel l'unité de surveillance (107) est en outre conçue pour évaluer l'état de santé de l'utilisateur en comparant les données enregistrées avec les données dérivées relatives à l'activité oculaire de l'utilisateur et les données environnementales dérivées.

9. Système (100) selon l'une quelconque des revendications précédentes, comprenant en outre :
une interface utilisateur (135) conçue pour recevoir une entrée de l'utilisateur, l'utilisateur indiquant si l'état de santé correspond aux données d'activité oculaire dérivées et/ou aux données environnementales dérivées.

10. Système (100) selon la revendication 9, dans lequel l'interface utilisateur (135) est en outre conçue pour indiquer si une anomalie physique et/ou psychologique s'est produite ou non sur la base de la comparaison.

11. Système (100) selon l'une quelconque des revendications précédentes, dans lequel les données enregistrées comprennent des données d'étalonnage précédemment stockées et des données environnementales précédemment stockées provenant de l'utilisateur et/ou d'autres utilisateurs.

12. Système (100) selon l'une quelconque des revendications précédentes, dans lequel l'unité de surveillance comprend en outre :
une unité d'alarme (130) conçue pour indiquer à un utilisateur qu'une anomalie physique et/ou psychologique s'est produite sur la base de la comparaison.
